# EUROPÄISCHE PATENTSCHRIFT

(11) **EP 0 954 343 B1**
(45) Veröffentlichungstag und Bekanntmachung des Hinweises auf die Patenterteilung: **01.09.2004**
(21) Anmeldenummer: 97954397.2
(22) Anmeldetag: 12.12.1997
(51) Int. Cl.: A61M 5/14

(54) **GERÄT ZUR INSTILLATION ODER INJEKTION VON ARZNEIMITTELN**
DEVICE FOR THE INSTILLATION OR INJECTION OF MEDICAMENTS
APPAREIL POUR L'INSTILLATION OU L'INJECTION DE MEDICAMENTS

(30) Priorität: 17.12.1996 DE 29621797 U; 17.01.1997 DE 29700746 U
(43) Veröffentlichungstag der Anmeldung: 10.11.1999
(73) Patentinhaber: STADA ARZNEIMITTEL AG, 61118 Bad Vilbel (DE)
(72) Erfinder: GIETMANN, Tobias, D-47533 Kleve (DE)
(74) Vertreter: Hamm, Volker, Dr.
(86) Internationale Anmeldenummer: PCT/EP1997/007003
(87) Internationale Veröffentlichungsnummer: WO 1998/026820

(56) Entgegenhaltungen:
- WO-A-95/01197
- US-A- 4 804 366
- US-A- 4 834 705

## Beschreibung

Die Erfindung betrifft ein Gerät zur Instillation von Arzneimitteln, insbesondere Zytostatika in Körperhöhlen mittels einer Kathetereinrichtung oder zur Injektion von Arzneimitteln, insbesondere Zytostatika, mit einer Aufnahme für einen das Arzneimittel enthaltenden Behälter.

Zytostatika sind Substanzen, die das Wachstum pathologischer Zellen, aber auch normaler Zellen, hemmen. Derartige Substanzen werden deshalb zur chemotherapeutischen Behandlung von Tumoren, aber auch zur postoperativen Behandlung nach Entfernung eines Tumors eingesetzt. Zytostatika können in flüssiger, pulverförmiger oder körniger Form, ggf. auch in tiefgefrorenen Zustand vorliegen. Weil eine Kontamination mit Zytostatika, zum Beispiel durch Hautkontakt, einatmen oder dgl., für gesunde Personen, insbesondere Personen, die mit Geräten der eingangs beschriebenen Gattung umgehen, gefährlich ist, werden diese Substanzen regelmäßig in verschlossenen Behältern, insbesondere in Fläschchen, die mit einem durch eine Metallbördelung befestigten Gummistopfen verschlossenen sind (Injektionsflasche), geliefert.

Ein aus der Praxis bekanntes Gerät der eingangs beschriebenen Gattung besteht aus einem Schlauchsystem mit einer in den Schlauch integrierten, ballonartigen oder tütenförmigen, mit einer Hand zu betätigenden Pumpe, die einerseits einen mit einem Ventil verschließbaren Anschluß für eine Kathetereinrichtung und andererseits einen Schlauchabschnitt mit einer Aufnahme für den das Zytostatikum enthaltenden Behälter sowie einen weiteren, mit einem Rückschlagventil versehenen Schlauchabschnitt für die Zuführung einer Flüssigkeit wie z. B. eine Salzlösung aufweist.

Die Aufnahme für den Behälter weist einen Aufnahmeboden mit einem daran angeordneten Hohldorn auf, der bei Einsetzen des Behälters in die Aufnahme den Gummistopfen durchsticht, so daß der Inhalt des Behälters durch den Hohldorn in das Schlauchsystem überführt werden kann. Im übrigen erstreckt sich die Aufnahme nur über einen geringen Teil der Behälterlänge, insbesondere nur über den mit der Metallbördelung versehenen Bereich einer Injektionsflasche, so daß es durch schiefes Aufsetzen oder Einsetzen des Behälters in die Aufnahme zum unerwünschten Austreten des Behälterinhaltes und damit zu einer Kontamination kommen kann. Außerdem muß darauf geachtet werden, daß der Behälter sich nicht aus der Aufnahme löst und dadurch das Zytostatikum austreten kann. Um den Inhalt des Behälters indas Schlauchsystem zu überführen, wird das zum Kathetereinrichtung führende Ventil geschlossen und die Pumpe betätigt. Aufgrund der damit erzeugten Druckänderungen wird das Zytostatikum in das Schlauchsystem überführt. Je nach Konsistenz des Zytostatikums muß der Behälter in unterschiedlichen räumlichen Positionen gehalten werden, damit ggf. die Flüssigkeit in den Behälter eingesaugt oder eingedrückt wird, um das Zytostatikum in der Flüssigkeit zu lösen bzw. es mit der Flüssigkeit zu mischen. Durch Halten des Behälters in einer anderen räumlichen Position kann die Lösung bzw. Mischung dann durch Pumpen in das Schlauchsystem überführt werden.

Die Druckschrift WO-A-95/01197 offenbart ein Gerät zur Instillation oder Injektion von Arzneimitteln mit einer Aufnahme für einen das Arzneimittel enthaltenen Behälter.

Aufgabe der Erfindung ist es, ein Gerät der eingangs beschriebenen Gattung anzugeben, das sich einfacher und sicherer handhaben läßt.

Diese Aufgabe wird durch die Merkmale im Kennzeichen des Anspruchs 1 gelöst.

Erfindungsgemäß wird eine Pumpe aus einem Zylinder, der an einem Ende offen ist und am anderen Ende einen Anschluß für eine Kathetereinrichtung aufweist, und aus dem im Zylinder bewegbaren, als sich bis über das offene Ende hinaus erstreckenden Hohlkolben als Aufnahme für den darin, vorzugsweise verriegelbaren Behälter gebildet, so daß bei diesem Gerät der Behälter zur Sicherung gegen versehentliches Lösen auch nicht an oder in der Aufnahme gehalten werden muß, weil er ist nach dem Einsetzen in den Hohlkolben dem unmittelbaren Zugriff entzogen ist und nur zusammen mit dem Hohlkolben bewegt werden kann. Die erforderliche Pumpwirkung zum Verlagern des Behälterinhaltes wird durch Bewegen des Hohlkolbens im Zylinder erzeugt.

Erfindungsgemäß weist der Hohlkolben zylinderseitig einen Boden mit einem im wesentlichen mittig angeordneten, sich in den Hohlkolben erstreckenden Hohldorn auf, der mit einem in den Zylinder mündenden Auslaß verbunden ist. Dementsprechend wird der Gummistopfen des Behälters erst dann durchstochen, wenn der Behälter in den Hohlkolben eingesetzt wird.

Zum Schutz des Hohldorns und zur besseren Führung der Behälteröffnung mit Stopfen kann der Hohldorn von einem Führungskragen für die Behälteröffnung umgeben sein.

Vorteilhafterweise kann der Führungskragen abgeschrägte, insbesondere konisch zulaufende Innenflanken aufweisen, durch die die Behälteröffnung beim Einsetzen selbsttätig in die richtige Position geführt wird.

Auch kann der Hohlkolben an seinem aus dem Zylinder ragenden Ende einen mit dem Hohlkolben verriegelbaren Deckel aufweisen, an den ein in den Hohlkolben ragender Stößel anschließt, zwischen dem und dem Boden des Hohlkolbens der Behälter einspannbar ist.

Insbesondere kann der Deckel als Schraubdeckel ausgebildet und in oder auf ein zugeordnetes Gewinde des Hohlkolbens bzw. des Aufbewahrungszylinders schraubbar sein, so daß der Behälter, nachdem er in den Hohlkolben eingeführt ist und der Deckel auf den Hohlkolben aufgesetzt wird, durch eine Schraubbewegung gegen den Boden des Hohlkolbens gedrückt und damit der Hohldorn durch den Gummistopfen gedrückt wird. Das Gewinde kann eine verhältnismäßig große Gewindesteigung aufweisen, so daß zum Beispiel eine einzige Umdrehung des Deckels genügt, um den Gummistopfen zu durchstoßen und den Behälter im Hohlkolben zu verriegeln.

Um den Deckel besser aufschrauben zu können und ferner eine Relativbewegung zwischen Zylinder und Hohlkolben zur Erzeugung einer Pumpwirkung zu erleichtern, kann der Dekkel außenseitig eine Handhabe aufweisen.

Im übrigen kann die Kathetereinrichtung geräteseitig in bekannter Weise ein Ventil aufweisen und zwischen dem Anschluß des Zylinders bzw. zwischen dem Aufbewahrungszylinder und dem Ventil der Kathetereinrichtung kann ein mit einem Rückschlagventil gesicherter Zulauf für eine Flüssigkeit angeordnet sein.

Vorteilhafterweise kann zwischen Hohldorn und Führungskragen ein erhabener, umlaufender Kranz ausgebildet sein, so daß eine Abdichtung des durch den Gummistopfen gedrückten Hohldorns erzielt wird.

Erfindungsgemäß kann das Gerät als geschlossenes System ausgebildet sein, so daß sowohl für den Patienten als auch für das das Arzneimittel, insbesondere Zytostatikum anwendende Personal die Gefahr eines versehentlichen äußeren Kontaktes mit dem Arzneimittel gänzlich ausgeschlossen ist und auch die Möglichkeit einer Verunreinigung des Arzneimittels vermieden wird.

Im folgenden werden in der Zeichnung dargestellte Ausführungsbeispiele der Erfindung erläutert; es zeigen:
- Fig. 1: einen Längsschnitt durch ein erstes Ausführungsbespiel,
- Fig. 2: einen anderen Längsschnitt des Gegenstandes nach Fig. 1.

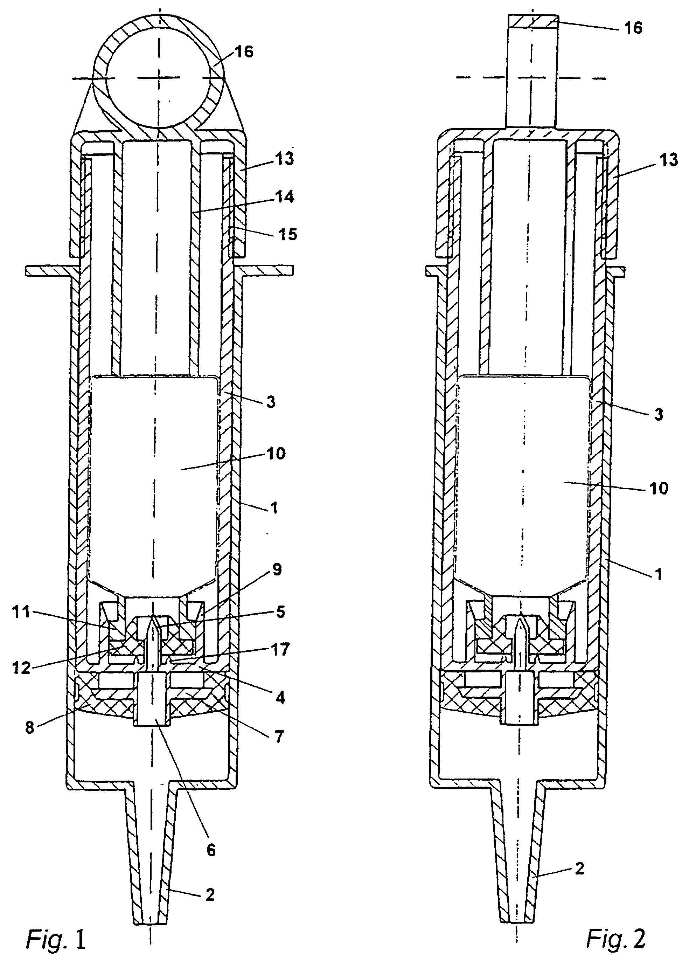

Zu dem in Fig. 1 dargestellten Gerät gehört ein langgestreckter Zylinder 1, der an einem Ende offen ist und am anderen Ende einen Anschluß 2 für einen nicht dargestellte Kathetereinrichtungeinrichtung aufweist. Im Zylinder 1 ist ein Hohlkolben 3 geführt, der sich bis über das offene Ende des Zylinders 1 hinaus erstreckt und der zylinderseitig einen Boden 4 aufweist. Mittig auf dem Boden 4 ist ein sich in den Hohlkolben 3 erstreckender Hohldorn 5 angeordnet, der mit einem in den Zylinder 1 mündenden stutzenartigen Auslaß 6 verbunden ist. Der Auslaß 6 ist von einem Kragen 7 umgeben, an dem eine mit der Innenseite des Zylinders 1 zusammenwirkende Dichtung 8 angeordnet ist. Der Hohldorn 5 ist von einem vom Boden 4 ausgehenden Führungskragen 9 mit abgeschrägten Innenflanken umgeben.

Ein Behälter 10 mit einer Behälteröffnung 11 ist in den Hohlkolben 3 eingesetzt. Der Behälter 10 enthält ein Zytostatikum, das flüssig, pulverförmig oder fest, ggf. gefriergetrocknet, sein kann. Die Behälteröffnung 11 greift in den Führungskragen 9 ein. Der Hohldorn 5 hat den Gummistopfen 12 durchstochen und wird durch einen erhabenen, umlaufenden Kranz 17, der gegen den Gummistopfen 12 drückt, abgedichtet.

Der Hohlkolben 3 weist an seinem aus dem Zylinder 1 ragenden Ende einen mit dem Hohlkolben 3 verriegelbaren Deckel 13 auf, an den sich ein in den Hohlkolben 3 ragender Stößel 14 anschließt, zwischen dem und dem Boden 4 des Hohlkolbens 3 der Behälter 10 eingespannt ist. Bei der dargestellten Ausführung ist der Deckel 13 als Schraubdeckel ausgebildet und auf ein zugeordnetes Gewinde 15 an der Außenseite des Hohlkolbens 3 aufgeschraubt. Das Gewinde 15 besitzt eine so große Steigung, daß praktisch mit einer Umdrehung des Deckels 13 ein in den Hohlkolben 3 eingesetzter Behälter 10 die in der Zeichnung wiedergegebene Funktionsstellung erreicht. Im übrigen weist der Deckel 13 außenseitig eine Handhabe 16 auf.

Nicht dargestellt ist, daß die an den Anschluß 2 anschließbare Kathetereinrichtungeinrichtung ein Ventil aufweist, und daß zwischen dem Anschluß 2 des Zylinders 1 und dem Ventil und der Kathetereinrichtungeinrichtung ein mit - einem Rückschlagventil gesicherter Zulauf für eine Flüssigkeit angeordnet ist.

Mit dem dargestellten Gerät wird wie folgt gearbeitet: bei abgenommenen Deckel 13 wird in den Hohlkolben 3 der mit dem Gummistopfen 12 verschlossene Behälter 10 eingesetzt. Dann wird der Deckel 13 auf den Hohlkolben 3 aufgesetzt und auf das Gewinde 15 aufgeschraubt. Dabei wird der Behälter 10 in Richtung auf den Boden 4 des Hohlkolbens 3 gedrückt, wobei der Hohldorn 5 den Gummistopfen 12 durchstößt. Danach ist der Behälter 10 im Hohlkolben 3 verriegelt. Bei geschlossenem Ventil der Kathetereinrichtung kann nunmehr durch Hin- und Herbewegung des Hohlkolbens 3 im Zylinder 1 eine Pumpwirkung mit Druckschwankungen erzeugt werden, die dazu führen, daß Flüssigkeit in den Behälter 10 gelangt und dessen Inhalt löst sowie in den Zylinderraum transportiert. Wenn der Zylinderraum mit der gewünschten Lösung gefüllt ist, wird das Ventil der Kathetereinrichtung geöffnet und die Lösung kann durch weiteres Einschieben des Hohlkolbens 3 in den Zylinder 1 in die Körperhöhle instilliert werden.

## Patentansprüche

1. Gerät zur Instillation oder Injektion von Arzneimitteln mit einer Aufnahme für einen das Arzneimittel enthaltenen Behälter (10), **dadurch gekennzeichnet, dass** es einen an einem Ende offenen und am anderen Ende mit einem Anschluss (2) versehenen Zylinder (1) sowie einen im Zylinder (1) bewegbaren Hohlkolben (3) umfasst, der als Aufnahme für den Behälter (10) ausgebildet ist und der zylinderseitig einen Boden (4) mit einem im wesentlichen mittig angeordneten, sich in den Hohlkolben (3) erstreckenden Hohldom (5) aufweist, der mit einem in den Zylinder (1) mündenden Auslass (6) verbunden ist.

2. Gerät nach Anspruch 1, **dadurch gekennzeichnet, dass** der Hohldom (5) von einem Führungskragen (9) für die Behälteröffnung (11) umgeben ist.

3. Gerät nach Anspruch 2, **dadurch gekennzeichnet, dass** der Führungskragen (9) abgeschrägte, insbesondere konisch zulaufende Innenflanken aufweist.

4. Gerät nach einem der Ansprüche 1 bis 3, **dadurch gekennzeichnet, dass** der Hohlkolben (3) an seinem aus dem Zylinder (1) ragenden Ende einen mit dem Hohlkolben (3) verriegelbaren Deckel (13) aufweist, an den ein in den Hohlkolben (3) ragender Stößel (14) anschließt, zwischen dem und dem Boden (4) des Hohlkolbens (3) der Behälter (10) einspannbar ist.

5. Gerät nach Anspruch 4, **dadurch gekennzeichnet, dass** der Deckel (13) als Schraubdeckel ausgebildet und in oder auf ein zugeordnetes Gewinde (15) des Hohlkolbens (3) schraubbar ist.

6. Gerät nach einem der Ansprüche 4 oder 5, **dadurch gekennzeichnet, dass** der Deckel (13) außenseitig eine Handhabe (16) aufweist.

7. Gerät nach einem Anspruch 1 bis 6, **dadurch gekennzeichnet, dass** es des weiteren eine Kathetereinrichtung umfasst.

8. Gerät nach Anspruch 7, **dadurch gekennzeichnet, dass** die Kathetereinrichtung geräteseitig ein Ventil aufweist.

9. Gerät nach Anspruch 8, **dadurch gekennzeichnet, dass** zwischen dem Anschluss (2) des Zylinders (1) und dem Ventil der Kathetereinrichtung ein mit einem Rückschlagventil gesicherter Zulauf für eine Flüssigkeit angeordnet ist.

10. Gerät nach einem der Ansprüche 2 bis 9, **dadurch gekennzeichnet, dass** zwischen Hohldom (5) und Führungskragen (9) ein erhabener, umlaufender Kranz (17) ausgebildet ist.

11. Gerät nach einem der Ansprüche 1 bis 10, **dadurch gekennzeichnet, dass** das Gerät als geschlossenes System ausgebildet ist.

## Claims

1. Apparatus for the instillation or injection of pharmaceutics, having a receiving means for a container (10) containing the pharmaceutics, **characterised in that** it comprises a cylinder (1) which is open at one end and is provided with a connection (2) at the other end and it also comprises a hollow piston (3) which can move in the cylinder (1), is formed as the receiving means for the container (10) and comprises a base (4) on the cylinder-side, said base having a hollow mandrel (5) which extends within the hollow piston (3), is disposed substantially in the centre and is connected to an outlet (6) issuing into the cylinder (1).

2. Apparatus as claimed in Claim 1, **characterised in that** the hollow mandrel (5) is surrounded by a guide collar (9) for the container opening (11).

3. Apparatus as claimed in Claim 2, **characterised in that** the guide collar (9) comprises inclined, in particular conically extending, inner surfaces.

4. Apparatus as claimed in any one of Claims 1 to 3, **characterised in that** the hollow piston (3) comprises at its end projecting from the cylinder (1) a cover (13) which can be locked to the hollow piston (3), a tappet (14) projects into the hollow piston (3) lying against this cover (13) and the container (10) can be clamped between the tappet (14) and the base (4) of the hollow piston (3).

5. Apparatus as claimed in Claim 4, **characterised in that** the cover (13) is formed as a screw cap and can be screwed in or on an associated thread (15) of the hollow piston (3).

6. Apparatus as claimed in any one of Claims 4 or 5, **characterised in that** the cover (13) comprises a handle (16) on the exterior.

7. Apparatus as claimed in any one of Claims 1 to 6, **characterised in that** it also comprises a catheter device.

8. Apparatus as claimed in Claim 7, **characterised in that** the catheter device comprises a valve on the apparatus-side.

9. Apparatus as claimed in Claim 8, **characterised in that** an inlet for a fluid which is secured to a check valve is disposed between the connection (2) of the cylinder (1) and the valve of the catheter device.

10. Apparatus as claimed in any one of Claims 2 to 9, **characterised in that** a convex, circumferential ring (17) is formed between the hollow mandrel (5) and the guide collar (9).

11. Apparatus as claimed in any one of Claims 1 to 10, **characterised in that** the apparatus is formed as a closed system.

## Revendications

1. Appareil pour l'installation ou l'injection de médicaments comportant un logement pour un réservoir (10) contenant le médicament, **caractérisé en ce qu'**il renferme un cylindre (1) ouvert sur une extrémité et qu'il est muni, à l'autre, d'un raccord (2) ainsi que d'un piston creux (3) mobile dans le cylindre (1), qui est configuré comme logement pour le réservoir (10) et qui présente, côté cylindre, un fond (4) comportant un goujon creux (5) disposé essentiellement au centre et s'étendant dans le piston creux (3), qui est relié à une évacuation (6) débouchant dans le cylindre (1).

2. Appareil selon la revendication 1, **caractérisé en ce que** le goujon creux (5) est entouré d'une collerette de guidage (9) pour l'ouverture du réservoir (11).

3. Appareil selon la revendication 2, **caractérisé en ce que** la collerette de guidage (9) présente en particulier des flancs intérieurs formant un cône.

4. Appareil selon l'une quelconque des revendications 1 à 3, **caractérisé en ce que** le piston creux (3) présente, sur son extrémité dépassant du cylindre (1) un couvercle (13) verrouillable avec le piston creux (3), couvercle auquel s'adjoint un poussoir (14) dépassant dans le piston creux (3), entre lequel et le fond (4) du piston creux (3) le réservoir (10) peut être inséré.

5. Appareil selon la revendication 4, **caractérisé en ce que** le couvercle (13) a la forme d'un couvercle à vis et dans lequel ou sur lequel un filetage conjugué (15) du piston creux (3) peut être vissé.

6. Appareil selon l'une quelconque des revendications 4 ou 5, **caractérisé en ce que** le couvercle (13) présente une prise (16) à l'extérieur.

7. Appareil selon l'une quelconque des revendications 1 à 6, **caractérisé en ce qu'**il renferme par ailleurs un dispositif de cathéter.

8. Appareil selon la revendication 7, **caractérisé en ce que** le dispositif de cathéter présente une soupape côté appareil.

9. Appareil selon la revendication 8, **caractérisé en ce que**, entre le raccord (2) du cylindre (1) et la soupape du dispositif de cathéter est disposée une arrivée pour un liquide sécurisée par un clapet anti-retour.

10. Appareil selon l'une quelconque des revendications 2 à 9, **caractérisé en ce que**, entre le goujon creux (5) et la collerette de guidage (9) est formée une couronne (17) périphérique en relief.

11. Appareil selon l'une quelconque des revendications 1 à 10, **caractérisée en ce que** l'appareil est configuré en système fermé.
